# EUROPÄISCHE PATENTSCHRIFT

(11) **EP 0 956 853 B1**
(45) Veröffentlichungstag und Bekanntmachung des Hinweises auf die Patenterteilung: **14.12.2005**
(21) Anmeldenummer: 99810383.2
(22) Anmeldetag: 04.05.1999
(51) Int. Cl.: A61K 9/107, A61K 9/48

(54) **Verwendung von Nanodispersionen in pharmazeutischen Endformulierungen**
Use of nanodispersions in pharmaceutical compositions
Utilisation des nanodispersions dans des compositions pharmaceutiques

(30) Priorität: 11.05.1998 EP 98810422
(43) Veröffentlichungstag der Anmeldung: 17.11.1999
(73) Patentinhaber: Ciba Specialty Chemicals Holding Inc., 4057 Basel (CH); VESIFACT AG, 6340 Baar (CH)
(72) Erfinder: Supersaxo, Andreas Werner, 6340 Baar (CH); Weder, Hans Georg, 8803 Rüschlikon (CH); Hüglin, Dietmar, 79591 Eimeldingen (DE); Röding, Joachim Friedrich, 79410 Badenweiler (DE)

(56) Entgegenhaltungen:
- EP-A- 0 349 150
- EP-A- 0 429 248
- EP-A- 0 711 556
- EP-A- 0 711 557
- WO-A-92/18147
- WO-A-93/02664
- WO-A-94/26252
- WO-A-96/13273
- WO-A-96/37192
- WO-A-97/21428
- US-A- 4 675 193
- US-A- 4 816 247
- US-A- 5 338 761
- US-A- 5 444 041
- US-A- 5 472 706
- US-A- 5 633 226
- US-A- 5 646 109
- US-A- 5 688 761
- PATENT ABSTRACTS OF JAPAN vol. 097, no. 007, 31.Juli 1997 & JP 09 059145 A (TAISHO PHARMACEUT CO LTD), 4.März 1997,

## Beschreibung

Die vorliegende Erfindung betrifft Nanodispersionen sowie deren Verwendung in pharmazeutischen Endformulierungen, pharmazeutische Endformulierungen, enthaltend diese Nanodispersionen sowie die verschiedenen pharmazeutischen Verwendungen dieser Endformulierungen.

Unter pharmazeutischen Endformulierungen werden hier solche Formulierungen verstanden, die neben ihren Grundstoffen, die für die Bildung der pharmazeutischen Formulierung verantwortlich sind, weitere funktionelle Wirksubstanzen enthalten. Diese werden den pharmazeutischen Grundformulierungen zugegeben und können zur therapeutischen Behandlung des Nervensystems, des endokrinen Systems, des Herz-Kreislaufsystems, des Respirationstrakts, des Magen-Darm-Kanals, der Niere und ableitenden Harnwege, des Bewegungsapparates, des Immunsystems, der Haut und Schleimhäute sowie zur Behandlung von Infektionskrankheiten dienen.

Damit diese Stoffe ihre Wirkung an der gewünschten Stelle entfalten können, müssen diese an den jeweiligen Wirkungsort gelangen. Um die Verfügbarkeit am Wirkort zu optimieren, werden zahlreiche Wirkstoffe mittels sogenannter Träger- und Transportvehikel (Carriersysteme) wie z.B. Mischmizellen, Liposomen oder Nanoemulsionen (Nanopartikel) appliziert. Beispiele solcher Wirkstoffe sind Amphotericin (NeXstar, Sequus, TLC), Daunorubicin (NeXstar), Doxorubicin (Sequus), inaktivierte Hepatitis A Viren (Berna), oder Econazol (Cilag). Applikation dieser Wirkstoffe mittels dieser Carriersysteme führt zu therapeutischen Vorteilen wie weniger Nebenwirkungen oder einer besseren Impfwirkung.

WO 96/13273 sowie US-4816247 beschreibten Emulsionspräkonzentrate enthaltend u.a. Ölphase, Emulgator und pharmazeutischen Wirkstoff, welche nach Verabreichung Mikroemulsionen bilden. US-5444041 lehrt in ähnlicher Weise die Herstellung einiger Wasser-in-Öl-Mikroemulsionen aus bestimmten Fettsäureglyceriden, Polyoxyethylen- und/oder Polyalkohol-modifizierten Fettsäuren und einer wässrigen Phase, welche sich nach Verabreichung in eine Öl-in-Wasser-Emulsion umwandeln. EP-A-349150 und US-5338761 lehren die Herstellung transparenter Öl-in-Wasser-Dispersionen durch Mischen einer Ölphase, eines oder mehrerer Emulgatoren und eines Polyalkohols unter Anwendung hoher Scherkräfte. WO 96/37192 beschreibt die Herstellung vom Nanodispersionen durch intensives Mischen einer Ölphase u.a. mit einem polyethoxylierten Sorbitanfettsäureester, einem Sphingolipid, einem Phospholipid und Ethanol. WO97/21428 offenbart ähnliche Zusammensetzungen als pharmazeutischen Wirkstoffträger.

Es wurden nun Formulierungen gefunden, welche die einfache Herstellung von Nanodispersionen mit sehr feiner Partikelgrösse erlauben, so dass in geeigneter Zusammensetzung die Wirksamkeit von Arzneistoffen in pharmazeutischen Endformulierungen erhöht werden kann.

Gegenstand der vorliegenden Erfindung ist daher eine Nanodispersion enthaltend
(a) 0,1 - 30 Gew-% eines Phospholipides,
(b) 1 - 50 Gew-%.von Polyethoxylierte. Fettalkohole, Polyethoxylierte Fettsäuren, Polyethoxylierte Vitamin E Derivate, Polyethoxyliertes Lanolin und dessen Derivate, Polyethoxylierte Fettsäurepartialglyceride, Polyethoxylierte Alkylphenole, Schwefelsäurehalbester polyethoxylierter Fettalkohole und deren Salze, Polyethoxylierte Fettamine und Fettsäureamide, Polyethoxylierte Kohlenhydrate, oder Blockpolymerisate von Ethylenoxid und Propylenoxid,
(c) 0,1 - 80 Gew-% eines natürlichen oder eines synthetischen oder eines partialsynthetischen Di- oder Triglycerid; eines Mineralöls, eines Silikonöls, eines Wachs, eines Fettalkohols, eines Guerbet-Alkohols oder dessen Ester, eines lipophilen funktionellen pharmazeutischen Wirkstoffes oder eine Mischung dieser Stoffe, und
(d) Ethanol,
welche erhältlich ist durch
(α) Mischen der Komponenten mit gewöhnlichen Rührapparaten (a), (b), (c) und (d) bis eine homogene, klare Flüssigkeit entsteht, und
(β) Zugabe der im Schritt (α) erhaltenen Flüssigkeit in die Wasserphase, dadurch gekennzeichnet, dass die Schritte (α) und (β) ohne hohen Scher- oder Kavitationskräfte erfolgen. Ebenso umfasst ist die Verwendung der Nanodispersion in pharmazeutischen Endformulierungen.

Die Nanodispersion ist erhältlich durch (α) Mischen der Komponenten (a), (b)(c) und (d), bis eine homogene, klare Flüssigkeit (Nanodispersionsvorphase) entsteht, und
β) Zugabe der im Schritt (α) erhaltenen Flüssigkeit in die Wasserphase der pharmazeutischen Endformulierungen, wobei die Schritte (α) und (β) ohne weiteren Energieeintrag erfolgen.

Der Schritt (α) erfolgt gewöhnlich bei Raumtemperatur, gegebenenfalls unter Erwärmung und unter Normaldruckbedingungen. Das Mischen geschieht mit gewöhnlichen Rührapparaten, wie z.B. Propeller-, Schrägblatt- oder Magnetrührwerken und ohne die Zuhilfenahme von speziellen mechanischen Rührhilfen.

Das Mischen der Komponenten (a), (b), (c) und (d) (= Schritt (α)) erfolgt in wasserfreiem Medium, d.h. der Zusatz von Wasser ist nicht notwendig.

Der Schritt (β) erfolgt durch Zugabe der im Schritt (α) erhaltenen Flüssigkeit, der Nanodispersions-Vorphase, in die Wasserphase der pharmazeutischen Endformulierungen. Durch die besondere Auswahl der Komponenten (a), (b), (c) und (d) entstehen dabei unmittelbar ultrafeine, monodisperse Nanodispersionen. Dabei kann auf eine Homogenisierung mittels Düsen-, Rotor-Stator- oder Ultraschallhomogenisatoren verzichtet werden, die normalerweise dazu dient, grob- oder zumindest heterodisperse Systeme in feine monodisperse Systeme zu überführen. Der Schritt (β) ist daher gekennzeichnet durch die Abwesenheit von hohen Scher- oder Kavitationskräften

Der Schritt (β) wird gewöhnlich bei Raumtemperatur durchgeführt, die im Bereich der jeweiligen Öl/Wasser- Phaseninversionstemperatur (PIT) liegt.

Die durch die Verfahrensschritte (α) und (β) charakterisierten Nanodispersionen weisen Partikel mit einem mittleren Durchmesser von <50 nm, typischerweise kleiner 30 nm, auf. Die Verteilung ist monodispers und gehorcht einer Gauss-Verteilung.

Vorzugsweise wird eine Nanodispersion verwendet, die
(a) als membranbildende Moleküle Substanzen, die geeignet sind, Zweischichtsysteme (sog. "Bilayer") auszubilden,
(b) als Coemulgatoren Substanzen, die bevorzugt O/W Strukturen ausbilden und
(c) als lipophile Komponente einen für pharmazeutische Präparate gebräuchlichen lipophilen Stoff.

Vorzugsweise enthält die Nanodispersion als Komponente (a) ein Phospholipid 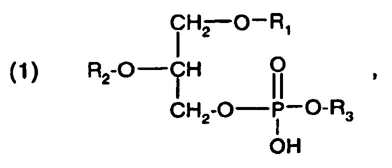 worin
- R₁: C₁₀-C₂₀-Acyl;
- R₂: Wasserstoff oder C₁₀-C₂₀-Acyl
- R₃: Wasserstoff, 2-Trimethylamino-1-ethyl, 2-Amino-1-ethyl, nicht substituiertes oder durch eine oder mehrere Carboxy-, Hydroxy- oder Amino-Gruppen substituiertes C₁-C₅-Alkyl; die Inositol- oder die Glycerylgruppe;
bedeuten; oder Salze dieser Verbindungen.

C₁₀-C₂₀-Acyl ist vorzugsweise geradkettiges C₁₀-C₂₀-Alkanoyl mit einer geraden Anzahl an C-Atomen und geradkettiges C₁₀-C₂₀-Alkenoyl mit einer Doppelbindung und einer geraden Anzahl an C-Atomen.

Geradkettiges C₁₀-C₂₀-Alkanoyl mit einer geraden Anzahl an C-Atomen sind beispielsweise n-Dodecanoyl, n-Tetradecanoyl, n-Hexadecanoyl oder n-Octadecanoyl.
Geradkettiges C₁₀-C₂₀-Alkenoyl mit einer Doppelbindung und einer geraden Anzahl an C-Atomen sind beispielsweise 6-cis- oder-6-trans-, 9-cis- oder 9-trans-Dodecenoyl, -Tetradecenoyl, -Hexadecenoyl, -Octadecenoyl oder -lcosenoyl, insbesondere 9-cis-Octadecenoyl (Oleoyl), femer 9,1 2-cis-Octadecadienoyl oder 9,12,15-cis-octadecatrienoyl.

Ein Phospholipid der Formel (1), worin R₃ 2-Trimethylamino-1-ethyl bedeutet, wird mit dem Trivialnamen Lecithin und ein Phospholipid der Formel (1), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen oder Mischungen davon.

Das Phospholipid der Formel (1) kann aber auch synthetischen Ursprungs sein. Unter dem Begriff synthetisches Phospholipid definiert man Phospholipide, welche bezüglich R₁ und R₂ eine einheitliche Zusammensetzung haben. Solche synthetischen Phospholipide sind vorzugsweise die weiter oben definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (Oleoyl).

Der Begriff "natürlich vorkommendes" Phospholipid definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ von natürlich vorkommenden Fettsäuregemischen abgeleitet sind.

Die Forderung "im wesentlichen reines" Phospholipid der Formel (1) definiert einen Reinheitsgrad von mehr als 90 Gew.-%, vorzugsweise mehr als 95 Gew.-% des Phospholipids der Formel (1), welcher anhand geeigneter Bestimmungsmethoden, z.B. papier- oder dünnschichtchromatographisch, mit HPLC oder enzymatischem Farbtest, nachweisbar ist.
In einem Phospholipid der Formel (1) ist R₃ mit der Bedeutung C₁-C₄-Alkyl beispielsweise Methyl oder Ethyl. Die Bedeutung Methyl ist bevorzugt.

R₃ mit den Bedeutungen durch eine oder mehrere Carboxy-, Hydroxy- oder Aminogruppen substituiertes C₁-C₅-Alkyl sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl, 3-Amino-3-carboxy-n-propyl oder 2-Amino-2-carboxy-n-propyl, vorzugsweise 2-Amino-2-carboxyethyl.

Phospholipide der Formel (1) mit diesen Gruppen können in Salzform, z.B. als Natrium- oder Kaliumsalz, vorliegen.

Phospholipide der Formel (1), worin R₃ die Inositol- oder die Glycerylgruppe bedeutet, sind unter den Bezeichnungen Phosphatidylinositol und Phosphatidylglycerol bekannt.

Für die Acylreste in den Phospholipiden der Formel (1) sind auch die in Klammern angegebenen Bezeichnungen gebräuchlich:
9-cis-Dodecenoyl (Lauroleoyl), 9-Cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 9,12-cis-Octadecadienoyl (Linoleoyl), 9,12,15-cis-Octadecatrienoyl (Linolenoyl), 11-cis-Octadecenoyl (Vaccenoyl), 9-cis-lcosenoyl (Gadoleoyl), 5,8,11,14-cis-Eicosatetraenoyl (Arachidonoyl), n-Dodecanoyl, (Lauroyl), n-Tetradecanoyl (Myristoyl), n-Hexadecanoyl (Palmitoyl), n-Octadecanoyl (Stearoyl), n-lcosanoyl (Arachidoyl), n-Docosanoyl (Behenoyl), n-Tetracosanoyl (Lignoceroyl).
Ein Salz des Phospholipids der Formel (1) ist vorzugsweise pharmazeutisch annehmbar. Salze definieren sich durch die Existenz von salzbildenden Gruppen im Substituenten R₃ sowie durch die freie Hydroxygruppe am Phosphoratom. Möglich ist ebenfalls die Bildung von inneren Salzen. Bevorzugt sind Alkalimetallsalze, insbesondere Natriumsalze.

In einer besonders bevorzugten Ausführungsform verwendet man gereinigtes Lecithin aus Sojabohnen der Qualität LIPOID S 100 oder S 75 oder ein Lecithin definiert in der Monographie USP23/NF 18.

Die Komponente (a) wird vorzugsweise in einer Konzentration von ca. 0,1 bis 30 Gew.-%, bezogen auf das Gesamtgewicht der Komponenten (a), (b) und (c), eingesetzt.

Als Komponente (b) wird vorzugsweise ein Emulgator oder Emulgatormischungen verwendet, der/die bevorzugt O/W-Strukturen ausbildet(n).

Speziell bevorzugte Emulgatoren sind
- Polyethoxylierte Fettalkohole wie z.B. Oleth-20;
- Polyethoxylierte Fettsäuren wie z.B. Polyoxyl 20 Stearat;
- Polyethoxyliertes Vitamin E Derivate wie z.B. Vitamin E Polyethylene Glycol 1000 Succinat;
- Polyethoxylierte Lanolin und Lanolin Derivate wie z.B. Laneth-20;
- Polyethoxylierte Fettsäurepartialglyceride wie z.B. Diethylenglykolmonostearat;
- Polyethoxylierte Alkylphenole wie z.B. Ethylphenolpoly(ethylenglykolether)11;
- Schwefelsäurehalbester polyethoxylierter Fettalkohole und deren Salze wie z.B. C₁₂-C₁₄-Fettalkoholethersulfat-2 EO-Natriumsalz;
- Polyethoxylierte Fettamine und Fettsäureamide;
- Polyethoxylierte Kohlenhydrate
- Blockpolymerisate von Ethylenoxid und Propylenoxid, wie z.B. Poloxamer 188.

Die Komponente (b) ist in der erfindungsgemäss verwendeten Nanodispersion in einer Konzentration von 1 bis 50 Gew.-%, bezogen auf das Gesamtgewicht der Komponenten (a), (b) und (c) vorhanden.

Die Komponente (c) ist ein natürliches oder ein synthetisches oder ein partialsynthetisches Di- oder Triglycerid, ein Mineralöl, ein Silikonöl, ein Wachs, ein Fettalkohol, ein Guerbet-Alkohol oder dessen Ester, ein lipophiler pharmazeutischer Wirkstoff oder eine Mischung dieser Stoffe.

Für die pharmazeutische Anwendung geeignete Wirkstoffe sind z.B. aus dem Arzneimittelkompendium 1997 zu entnehmen. Z.B. kommen in Betracht:
Analgetika, Antazida/Ulkusbehandlung, Antiallergika, Antianämika, Antidepressiva, Antidiabetika, Antidiarrhoika, Antidote/Entwöhnungsmittel/Emetika, Antiemetika/Antivertiginosa, Antiepileptika, Antihämorrhagika, Antihypertensiva, Antihypotonica, Antiinfektiva, Antikoagulantien, Antirheumatika/Entzündungshemmer, Appetitzügler, Betarezeptorenblocker, Bronchodilatantien, Cholinergika, Dermatologika, Desinfizientia, Diagnostika, Diätetika, Diuretika, durchblutungsfördernde Mittel, Gastroenterologika, Gichtmittel, Grippemittel, Gynäkologika, Hämorrhoidenmittel, Hormone, Hustenmittel, Hypnotika, Immunologika, Infundibilia, Kardiaka, Kontrazeptiva, Kontrastmittel, Kortikosteroide, Laxantien, Leber- und Gallentherapeutika, Lipidstoffwechsel, Lokalanästhetika, Migränemittel, Mineralstoffwechsel-Präparate, Muskelrelaxantien, Narkosemittel, Neuroleptika, Odontologika, Ophthalmika, Otorhinolaryngologie (ORL), Parkinson-Therapeutika, Psychostimulantien, Sedativa, Spasmolytika, Tonika/Roborantien, Tranquilizer, Tuberkulose-Therapeutika, Urologika, Venenmittel, Wundheilmittel und Zytostatica.

Die Komponente (c) ist in der erfindungsgemäss eingesetzten Nanodispersion in einer Konzentration von 0,1 bis 80 Gew.-%, bezogen auf das Gesamtgewicht der Komponenten (a), (b) und (c) vorhanden.
Die erfindungsgemäss eingesetzte Nanodispersion enthält als zusätzliche Komponente (d) einen Lösungsvermittler, Ethanol.

Eine Nanodispersionszusammensetzung mit den Komponenten (a), (b) und (c) und (d) zeichnet sich durch günstige Phaseneigenschaften aus. So ist bei vorhandener Opaleszenz und Transparenz im Gegenlicht nur an einer äusserst geringen milchigen Trübung zu erkennen, dass die Dispersion noch physikalische Unterschiede gegenüber dem Idealzustand einer echten molekularen Lösung aufweist. Elektronenmikroskopische Abbildungen zeigen, dass eine Population von mehr als 98 % in einer Gaussschen Verteilung als Suspension von Partikeln (Nanopartikel) mit einer Teilchengrösse kleiner als ca. 50 nm (Nanodispersion), typischerweise kleiner als ca. 30 nm, vorliegt. Diese Unterschiede gegenüber einer echten Lösung sind aber aufgrund der besonders guten Homogenitätseigenschaften der Dispersion tolerierbar, die beispielsweise an einer überraschend hohen Lagerstabilität, z. B. keine Entmischung nach mehrmonatiger Lagerung bei Temperaturen bis Raumtemperatur (durch Extrapolation zu erwartende Stabilität länger als zwei Jahre), nachweisbar sind.

Laser-Lichtstreumessungen und elektronenmikroskopische Untersuchungen (Cryo-TEM) bestätigen die sehr kleine Grösse und hervorragende Homogenität der in der Nanodispersion vorhandenen Nanopartikel.

Ein weiterer Vorteil der erfindungsgemäss verwendeten Nanodispersionen ist ihre einfache Herstellbarkeit.

Die oben charakterisierten Nanodispersionen werden erfindungsgemäss für pharmazeutische Endformulierungen verwendet.

Einen weiteren Erfindungsgegenstand bildet die durch den im Schritt (α) charakterisierte sogenannte "Nanodispersions-Vorphase", die erhältlich ist durch Mischen der Komponenten
(a) 0,1 -30 Gew-% eines Phospholipid,
(b) 1 - 50 Gew-% von Polyethoxylierte Fettalkohole, Polyethoxylierte Fettsäuren, Polyethoxylierte Vitamin E Derivate, Polyethoxyliertes Lanolin und dessen Derivate, Polyethoxylierte Fettsäurepartialglyceride, Polyethoxylierte Alkylphenole, Schwefelsäurehalbester polyethoxylierter Fettalkohole und deren Salze, Polyethoxylierte Fettamine und Fettsäureamide, Polyethoxylierte Kohlenhydrate, oder Blockpolymerisate von Ethylenoxid und Propylenoxid,
(c) 0,1 - 80 Gew-% eines natürlichen oder eines synthetischen oder eines partialsynthetischen Di- oder Triglycerid, eines Mineralöls, eines Silikonöls, eines Wachs, eines Fettalkohols, eines Guerbet-Alkohols oder dessen Ester, eines lipophilen funktionellen pharmazeutischen Wirkstoffes oder eine Mischung dieser Stoffe, und
(d) Ethanol,
bis eine homogene, klare Flüssigkeit entsteht, dadurch gekennzeichnet, dass das Mischen in wasserfreiem Medium erfolgt.

Die Nanodispersions-Vorphase oder die Nanodispersion wird erfindungsgemäss für pharmazeutische Endformulierungen verwendet.

Vorzugsweise sind die pharmazeutischen Endformulierungen flüssige, halbfeste oder feste Zubereitungen.

Beispiele flüssiger pharmazeutischer Endformulierungen sind Injektionslösungen, Infusionslösungen, Tropfen, Sprays, Aerosole, Emulsionen, Lotionen, Suspensionen, Trinklösungen, Gurgelwässer und Inhalate.

Beispiele halbfester pharmazeutischer Endformulierungen sind Salben, Cremes (O/W-Emulsionen), Fettcremes (W/O-Emulsionen), Gele, Lotionen, Schäume, Pasten, Suspensionen, Ovula, Pflaster einschliesslich transdermaler Systeme.

Beispiele fester pharmazeutischer Endformulierungen sind Tabletten, Dragees, Kapseln, Granulate, Brausegranulate, Brausetabletten, Pastillen, Lutsch- und Kautabletten, Arzneistoffstäbchen, Implantate, Lyophilisate, Adsorbate, Pulver oder Puder.

Diese Endformulierungen bilden einen weiteren Erfindungsgegenstand.

Die Endformulierungen enthalten dabei die Nanodispersion in einer Konzentration von 0,01 bis 100, vorzugsweise 0,05 bis 20, und insbesondere von 0,1 bis 10 Gew.-%.

Zur Herstellung von flüssigen und halbfesten pharmazeutischen Endprodukten werden die Nanodispersionen in den wässrigen Anteil des Endproduktes eingearbeitet. Anstelle der Nanodispersion kann auch die entsprechende Nanodispersionsvorphase der Wasserphase der pharmazeutischen Endformulierung zugegeben werden. Die Zugabe der Nanodispersions-Vorphase zur Wasserphase erfolgt unter Rühren und vorzugsweise bei einer Temperatur, die im Bereich der jeweiligen Öl/Wasser-Phaseninversionstemperatur (PIT) liegt.

Feste pharmazeutische Endprodukte wie z.B. Tabletten, Brausetabletten, Dragées, Tabletten mit Überzug, Granulate, Brausegranulate und Pflaster werden durch Besprühen oder Tränken mit Nanodispersionen überzogen oder beladen. In gewissen Fällen ist es vorteilhaft, die dehydratisierte Form der Nanodispersion dem festen Stoffgemisch beizumischen. Die Dehydratisierung der Nanodispersion erfolgt im allgemeinen durch Gefrier- oder Sprühtrocknung in Gegenwart üblicher Hilfsstoffe. Kapseln, insbesondere Weichgelatine-Kapseln können auch mit der Nanodispersions-Vorphase beladen werden.

Matrix- oder membrankontrollierte pharmazeutische Applikationssysteme wie z.B. Oros-Kapseln, transdermale Systeme, injizierbare Mikrokapseln oder Implantate werden mittels gängiger Methoden mit Nanodispersionen beschickt. Oros-Kapseln können auch mit der Nanodispersions-Vorphase beschickt werden.

Die erfindungsgemässe pharmazeutische Endformulierung kann neben den arzneiformgebenden Hilfsstoffen auch weitere Komponenten wie z.B. Stabilisatoren, Konservierungsmittel wie z.B. Parabene, Antioxidantien, sowie Aroma- Duft- oder Farbstoffe enthalten.
Die pharmazeutischen Endformulierungen werden vorzugsweise zur therapeutischen Behandlung des Nervensystems, des endokrinen Systems, des Herz-Kreislaufsystems, des Respirationstrakts, des Magen-Darm-Kanals, der Niere und ableitenden Harnwege, des Bewegungsapparates, des Immunsystems, der Haut und Schleimhäute sowie zur Behandlung von Infektionskrankheiten, Tumoren und Vitamin-/ Mineralstoffmangelkrankheiten verwendet.

Die erfindungsgemässe pharmazeutische Endformulierung wird dabei vorzugsweise epikutan, bukkal, lingual, sublingual, enteral (= peroral), rektal, nasal, pulmonal, per inhalationem, konjunktival, intravaginal, intraurethral, intrakardial, intraarteriell, intravenös, intralumbal, intrathekal, intraartikulär, intrakutan, subkutan, intramuskulär und intraperitoneal appliziert.

In den folgenden Beispielen beziehen sich die Prozentsätze auf das Gewicht. Die Mengen beziehen sich bei den eingesetzten Verbindungen, wenn nicht anders angegeben, auf die Reinsubstanz.

Herstellungsbeispiele für Nanodispersions-Vorphasen

### Beispiel 1: Miglyol 812 Nanodispersions -Vorphase

| | |
|---|---|
| Soja-Lecithin | 17,30 % |
| Oleth-20 | 34,00 % |
| Miglyol 812 | 34,50 % |
| Ethanol | 14,20 % |

Herstellung: Miglyol 812 und Oleth-20 werden unter Erwärmen gemischt. Zu dieser Mischung gibt man das in Ethanol gelöste Soja-Lecithin hinzu und erhält eine homogene, klare Flüssigkeit.

### Beispiel 2: Miglyol 812 Nanodispersions -Vorphase

| | |
|---|---|
| Soja-Lecithin | 17,30 % |
| Laneth-20 | 34,00 % |
| Miglyol 812 | 34,50 % |
| Ethanol | 14,20 % |

Herstellung: Miglyol 812 und Laneth-20 werden, unter Erwärmen gemischt. Zu dieser Mischung gibt man das in Ethanol gelöste Soja-Lecithin hinzu und erhält eine homogene, klare Flüssigkeit.

### Beispiel 3: Miglyol 812 Nanodispersions -Vorphase

| | |
|---|---|
| Soja-Lecithin | 17,30% |
| Vitamin E Polyethylene Glycol Succinate (Vitamin E TPGS, Eastman) | 34,00 % |
| Miglyol 812 | 34,50 % |
| Ethanol | 14,20 % |

Herstellung: Miglyol 812 und Vitamin E Polyethylene Glycol Succinate werden unter Erwärmen gemischt. Zu dieser Mischung gibt man das in Ethanol gelöste Soja-Lecithin hinzu und erhält eine homogene, klare Flüssigkeit.

### Herstellungsbeispiele für Nanodispersionen

### Beispiel 4: Miglyol 812 Nanodispersion

| | |
|---|---|
| Soja-Lecithin | 1,73 % |
| Oleth-20 | 3,40 % |
| Miglyol 812 | 3,45 % |
| Ethanol | 1,42 % |
| 10 mM Phosphatpuffer, pH 6 | ad 100,00 % |

Herstellung: Die Wasser-Phase (z.B. 90 kg) wird unter Rühren (z.B. Magnetrührerwerk) bei 50°C vorgelegt. Die flüssige Nanodispersions-Vorphase des Beispiels 1 (z.B. 10 kg) wird unter Rühren (z.B. mit einem Magnetrührwerk) der Wasserphase zugegeben.

### Beispiel 5: Migylol 812 Nanodispersion

| | |
|---|---|
| Soja-Lecithin | 1,73 % |
| Vitamin E Polyethylene Glycol Succinate (Vitamin E TPGS, Eastman) | 3,40 % |
| Miglyol 812 | 3,45 % |
| Ethanol | 1,42 % |
| 10 mM Phosphatpuffer, pH 6 | ad 100,00 % |

Die Herstellung der Nanodispersion erfolgt in zum Beispiel 4 analoger Weise.

### Beispiel 6: Miglyol 812 Nanodispersion

| | |
|---|---|
| Soja-Lecithin | 1,73 % |
| Vitamin E Polyethylene Glycol Succinate (Vitamin E TPGS, Eastman) | 3,40 % |
| Miglyol 812 | 3,45 % |
| Ethanol | 1,42 % |
| 10 mM Phosphatpuffer, pH 6 | ad 100,00 % |

Die Herstellung der Nanodispersion erfolgt in zum Beispiel 4 analoger Weise.

## Patentansprüche

1. Nanodispersion, enthaltend
(a) 0,1 - 30 Gew-% eines Phospholipid,
(b) 1 - 50 Gew-% von Polyethoxylierte Fettalkohole, Polyethoxylierte Fettsäuren, Polyethoxylierte Vitamin E Derivate, Polyethoxyliertes Lanolin und dessen Derivate, Polyethoxylierte Fettsäurepartialglyceride, Polyethoxylierte Alkylphenole, Schwefelsäurehalbester polyethoxylierter Fettalkohole und deren Salze, Polyethoxylierte Fettamine und Fettsäureamide, Polyethoxylierte Kohlenhydrate, oder Blockpolymerisate von Ethylenoxid und Propylenoxid,
(c) 0,1 - 80 Gew-% eines natürlichen oder eines synthetischen oder eines partialsynthetischen Di- oder Triglycerid, eines Mineralöls, eines Silikonöls, eines Wachs, eines Fettalkohols, eines Guerbet-Alkohols oder dessen Ester, eines lipophilen funktionellen pharmazeutischen Wirkstoffes oder eine Mischung dieser Stoffe, und
(d) Ethanol
erhältlich durch
(α) Mischen der Komponenten mit gewöhnlichen Rührapparaten (a), (b), (c) und (d) bis eine homogene, klare Flüssigkeit entsteht, und
(β) Zugabe der im Schritt (α) erhaltenen Flüssigkeit in die Wasserphase, **dadurch gekennzeichnet, dass** die Schritte (α) und (β) ohne hohen Scher- oder Kavitationskräfte erfolgen.

2. Pharmazeutische flüssige Endformulierung in Form einer Injektionslösung, einer Infusionslösung, von Tropfen, eines Sprays, eines Aerosols, einer Emulsion, einer Lotion, einer Suspension, einer Trinklösung, eines Gurgelwassers oder Inhalats, enthaltend eine Nanodispersion wie in Anspruch 1 definiert.

3. Pharmazeutische halbfeste Endformulierung in Form einer Salbe, einer Creme (O/W-Emulsionen), einer Fettcreme (W/O-Emulsionen), eines Gels, einer Lotion, eines Schaums, einer Paste, einer Suspension, eines Ovulas oder eines Pflasters, enthaltend eine Nanodispersion wie in Anspruch 1 definiert.

4. Pharmazeutische feste Endformulierung in Form einer Tablette, eines Dragées, einer Kapsel, eines Granulates, eines Brausegranulates, einer Brausetablette, einer Pastille, einer Lutsch- und Kautablette, eines Arzneistoffstäbchens, eines Implantates, eines Lyophilisates, eines Adsorbates, eines Pulvers oder Puders, enthaltend eine Nanodispersion wie in Anspruch 1 definiert.

5. Pharmazeutische Endformulierung nach einem der Ansprüche 2 oder 3, **dadurch gekennzeichnet, dass** die Nanodispersion in ihrer wässrigen Phase vorhanden ist.

6. Pharmazeutische Endformulierung nach einem der Ansprüche 2, 3 oder 5, **dadurch gekennzeichnet, dass** die wässrige Phase die Nanodispersion in einer Konzentration von 0,01 bis 100 Gew.-% enthält.

7. Pharmazeutische Endformulierung nach Anspruch 4, **dadurch gekennzeichnet, dass** die Nanodispersion per se vorhanden ist.

8. Pharmazeutische Endformulierung nach Anspruch 4, **dadurch gekennzeichnet, dass** die Nanodispersion in dehydratisierter Form vorliegt.

9. Nanodispersions-Vorphase, erhältlich durch Mischen der Komponenten
(a) 0,1 - 30 Gew-% eines Phospholipid,
(b) 1 - 50 Gew-% von Polyethoxylierte Fettalkohole, Polyethoxylierte Fettsäuren, Polyethoxylierte Vitamin E Derivate, Polyethoxyliertes Lanolin und dessen Derivate, Polyethoxylierte Fettsäurepartialglyceride, Polyethoxylierte Alkylphenole, Schwefelsäurehalbester polyethoxylierter Fettalkohole und deren Salze, Polyethoxylierte Fettamine und Fettsäureamide, Polyethoxylierte Kohlenhydrate, oder Blockpolymerisate von Ethylenoxid und Propylenoxid,
(c) 0,1 - 80 Gew-% eines natürlichen oder eines synthetischen oder eines partialsynthetischen Di- oder Triglycerid, eines Mineralöls, eines Silikonöls, eines Wachs, eines Fettalkohols, eines Guerbet-Alkohols oder dessen Ester, eines lipophilen funktionellen pharmazeutischen Wirkstoffes oder eine Mischung dieser Stoffe, und
(d) Ethanol
bis eine homogene, klare Flüssigkeit entsteht, **dadurch gekennzeichnet, dass** das Mischen in wasserfreiem Medium erfolgt.

10. Nanodispersions-Vorphase nach Anspruch 9, **dadurch gekennzeichnet, dass** das Mischen mit gewöhnlichen Rührapparaten ohne hohen Scher- oder Kavitationskräfte erfolgt

11. Verwendung einer Nanodispersion, enthaltend
(a) 0,1 - 30 Gew-% eines Phospholipid,
(b) 1 - 50 Gew-% von Polyethoxylierte Fettalkohole, Polyethoxylierte Fettsäuren, Polyethoxylierte Vitamin E Derivate, Polyethoxyliertes Lanolin und dessen Derivate, Polyethoxylierte Fettsäurepartialglyceride, Polyethoxylierte Alkylphenole, Schwefelsäurehalbester polyethoxylierter Fettalkohole und deren Salze, Polyethoxylierte Fettamine und Fettsäureamide, Polyethoxylierte Kohlenhydrate, oder Blockpolymerisate von Ethylenoxid und Propylenoxid,
(c) 0,1 - 80 Gew-% eines natürlichen oder eines synthetischen oder eines partialsynthetischen Di- oder Triglycerid, eines Mineralöls, eines Silikonöls, eines Wachs, eines Fettalkohols, eines Guerbet-Alkohols oder dessen Ester, eines lipophilen funktionellen pharmazeutischen Wirkstoffes oder eine Mischung dieser Stoffe, und
(d) Ethanol
in pharmazeutischen Endformulierungen, wobei die Nanodispersion erhältlich ist durch
(α) Mischen der Komponenten mit gewöhnlichen Rührapparaten (a), (b), (c) und (d) bis eine homogene, klare Flüssigkeit entsteht, und
(β) Zugabe der im Schritt (α) erhaltenen Flüssigkeit in die Wasserphase der pharmazeutischen Endformulierungen, **dadurch gekennzeichnet, dass** die Schritte (α) und (β) ohne hohen Scher- oder Kavitationskräfte erfolgen.

12. Verwendung nach Anspruch 11, **dadurch gekennzeichnet, dass** der Schritt (α) im wasserfreien Medium durchgeführt wird.

13. Verwendung nach Anspruch 11 oder 12, **dadurch gekennzeichnet, dass**, dass der Schritt (β) ohne Homogenisierung erfolgt.

14. Verwendung nach einem der Ansprüche 1 bis 13, **dadurch gekennzeichnet, dass** die in der Nanodispersion vorliegenden Partikel einen mittleren Durchmesser von <50 nm aufweisen.

15. Verwendung nach einem der Ansprüche 1 bis 14, **dadurch gekennzeichnet, dass** die pharmazeutische Endformulierung eine flüssige, halbfeste oder feste Zubereitung darstellt.

## Claims

1. A nanodispersion comprising
(a) 0.1 - 30% by weight of a phospholipid,
(b) 1 - 50% by weight of polyethoxylated fatty alcohols, polyethoxylated fatty acids, polyethoxylated vitamin E derivatives, polyethoxylated lanolin and the derivatives thereof, polyethoxylated fatty acid partial glycerides, polyethoxylated alkylphenols, sulfuric acid semiesters of polyethoxylated fatty alcohols and the salts thereof, polyethoxylated fatty amines and fatty acid amides, polyethoxylated carbohydrates, or block polymers of ethylene oxide and propylene oxide,
(c) 0.1 - 80% by weight of a natural or synthetic or a partially synthetic di- or triglyceride, mineral oil, silicone oil, wax, fatty alcohol, Guerbet alcohol or the ester thereof, a lipophilic functional pharmaceutical active ingredient or a mixture of these substances, and
(d) ethanol
and obtainable by
(α) mixing the components (a), (b), (c) and (d) with conventional stirring apparatus until a homogeneous clear liquid is obtained, and
(β) adding the liquid obtained in step (α) to the water phase wherein steps (α) and (β) are carried out without high shear or cavitation forces.

2. A pharmaceutical liquid end formulation in the form of an injectable solution, infusion solution, drops, spray, aerosol, emulsion, lotion, suspension, drinking solution, gargle or inhalant, comprising a nanodispersion as defined in claim 1.

3. A pharmaceutical semisolid end formulation in the form of an ointment, cream (O/W emulsions), rich cream (W/O emulsions), gel, lotion, foam, paste, suspension, ovula or plaster, comprising a nanodispersion as defined in claim 1.

4. A pharmaceutical solid end formulation in the form of a tablet, coated tablet, capsule, granules, effervescent granules, effervescent tablet, lozenge, pastille, medicament rod or stick, implant, lyophilisate, adsorbate or fine or coarse powder, comprising a nanodispersion as defined in claim 1.

5. A pharmaceutical end formulation according to either claim 2 or claim 3, wherein the nanodispersion is present in its aqueous phase.

6. A pharmaceutical end formulation according to any one of claims 2, 3 and 5, wherein the aqueous phase contains the nanodispersion in a concentration of 0.01 to 100% by weight.

7. A pharmaceutical end formulation according to claim 4, wherein the nanodispersion is present per se.

8. A pharmaceutical end formulation according to claim 4, wherein the nanodispersion is present in dehydrated form.

9. A nanodispersion prephase obtainable by mixing the components
(a) 0.1 - 30% by weight of a phospholipid,
(b) 1 - 50% by weight of polyethoxylated fatty alcohols, polyethoxylated fatty acids, polyethoxylated vitamin E derivatives, polyethoxylated lanolin and the derivatives thereof, polyethoxylated fatty acid partial glycerides, polyethoxylated alkylphenols, sulfuric acid semiesters of polyethoxylated fatty alcohols and the salts thereof, polyethoxylated fatty amines and fatty acid amides, polyethoxylated carbohydrates, or block polymers of ethylene oxide and propylene oxide,
(c) 0.1 - 80% by weight of a natural or synthetic or a partially synthetic di- or triglyceride, mineral oil, silicone oil, wax, fatty alcohol, Guerbet alcohol or the ester thereof, a lipophilic functional pharmaceutical active ingredient or a mixture of these substances, and
(d) ethanol
until a homogeneous clear liquid is obtained, wherein said mixing is carried out in an anhydrous medium.

10. A nanodispersion prephase according to claim 9, wherein said mixing is carried out with conventional stirring apparatus without high shear or cavitation forces.

11. The use of a nanodispersion comprising
(a) 0.1 - 30% by weight of a phospholipid,
(b) 1 - 50% by weight of polyethoxylated fatty alcohols, polyethoxylated fatty acids, polyethoxylated vitamin E derivatives, polyethoxylated lanolin and the derivatives thereof, polyethoxylated fatty acid partial glycerides, polyethoxylated alkylphenols, sulfuric acid semiesters of polyethoxylated fatty alcohols and the salts thereof, polyethoxylated fatty amines and fatty acid amides, polyethoxylated carbohydrates, or block polymers of ethylene oxide and propylene oxide,
(c) 0.1 - 80% by weight of a natural or synthetic or a partially synthetic di- or triglyceride, mineral oil, silicone oil, wax, fatty alcohol, Guerbet alcohol or the ester thereof, a lipophilic functional pharmaceutical active ingredient or a mixture of these substances, and
(d) ethanol
in a pharmaceutical end formulation, the nanodispersion being obtainable by
(α) mixing the components (a), (b), (c) and (d) until a homogeneous clear liquid is obtained, and
(β) adding the liquid obtained in step (α) to the water phase of the pharmaceutical end formulation, wherein steps (α) and (β) are carried out without high shear or cavitation forces.

12. The use according to claim 11, wherein step (α) is carried out in an anhydrous medium.

13. The use according to claim 11 or 12, wherein step (β) is carried out without homogenization.

14. The use according to any one of claims 11 to 13, wherein the particles in the nanodispersion have an average diameter of <50 nm.

15. The use according to any one of claims 11 to 14, wherein the pharmaceutical end formulation is a liquid, semisolid or solid preparation.

## Revendications

1. Nanodispersion contenant
(a) 0,1% à 30% en poids d'un phospholipide,
(b) 1% à 50% en poids d'alcools gras polyéthoxylés, d'acides gras polyéthoxylés, de dérivés de vitamine E polyéthoxylée, de lanoline polyéthoxylée et de ses dérivés, de glycérides partiels d'acides gras polyéthoxylés, d'alkylphénols polyéthoxylés, d'hémi-esters d'acide sulfurique et d'alcools gras polyéthoxylés et de leurs sels, d'amines grasses et d'amides gras polyéthoxylés, d'hydrates de carbone polyéthoxylés, ou de polymères séquencés d'oxyde d'éthylène et d'oxyde de propylène,
(c) 0,1% à 80% en poids d'un di- ou tri-glycéride naturel ou synthétique ou partiellement synthétique, d'une huile minérale, d'une huile de silicone, d'une cire, d'un alcool gras, d'un alcool de Guerbet ou de son ester, d'une substance active pharmaceutique fonctionnelle lipophile ou d'un mélange de ces substances, et
(d) de l'éthanol,
laquelle peut être obtenue
(α) en mélangeant les composants (a), (b), (c) et (d) dans des agitateurs habituels jusqu'à ce qu'il se forme un liquide transparent homogène, et
(β) en ajoutant le liquide obtenu à l'étape (α) dans la phase aqueuse, **caractérisée en ce que** les étapes (α) et (β) sont réalisées sans l'application de grandes forces de cisaillement ou de cavitation.

2. Formulation pharmaceutique finale liquide sous la forme d'une solution pour préparation injectable, d'une solution pour perfusion, de gouttes, d'une pulvérisation, d'un aérosol, d'une. émulsion, d'une lotion, d'une suspension, d'une solution buvable, d'un gargarisme ou d'un produit pour inhalation, contenant une nanodispersion telle que définie selon la revendication 1.

3. Formulation pharmaceutique finale semi-solide sous la forme d'une pommade, d'une crème (émulsions huile-dans-eau), d'une crème grasse (émulsions eau-dans-huile), d'un gel, d'une lotion, d'une mousse, d'une pâte, d'une suspension, d'un ovule ou d'un pansement pelliculaire, contenant une nanodispersion telle que définie selon la revendication 1.

4. Formulation pharmaceutique finale solide sous la forme d'un comprimé, d'un comprimé dragéifié, d'une capsule, d'un granulé, d'un granulé effervescent, d'un comprimé effervescent, d'une pastille, d'un comprimé à sucer ou à mâcher, d'un bâtonnet médicamenteux, d'un implant, d'un lyophilisat, d'un adsorbat, d'une poudre ou d'une poudre fine, contenant une nanodispersion telle que définie selon la revendication 1.

5. Formulation pharmaceutique finale selon l'une quelconque des revendications 2 ou 3, **caractérisée en ce que** la nanodispersion se trouve dans la phase aqueuse.

6. Formulation pharmaceutique finale selon l'une quelconque des revendications 2, 3 ou 5, **caractérisée en ce que** la phase aqueuse contient la nanodispersion dans une concentration de 0,01% à 100% en poids.

7. Formulation pharmaceutique finale selon la revendication 4, **caractérisée en ce que** la nanodispersion est présente en soi.

8. Formulation pharmaceutique finale selon la revendication 4, **caractérisée en ce que** la nanodispersion se trouve sous forme déshydratée.

9. Préphase de nanodispersion, pouvant être obtenue en mélangeant les composants
(a) 0,1% à 30% en poids d'un phospholipide,
(b) 1% à 50% en poids d'alcools gras polyéthoxylés, d'acides gras polyéthoxylés, de dérivés de vitamine E polyéthoxylée, de lanoline polyéthoxylée et de ses dérivés, de glycérides partiels d'acides gras polyéthoxylés, d'alkylphénols polyéthoxylés, d'hémi-esters d'acide sulfurique et d'alcools gras polyéthoxylés et de leurs sels, d'amines grasses et d'amides gras polyéthoxylés, d'hydrates de carbone polyéthoxylés, ou de polymères séquencés d'oxyde d'éthylène et d'oxyde de propylène,
(c) 0,1% à 80% en poids d'un di- ou tri-glycéride naturel ou synthétique ou partiellement synthétique, d'une huile minérale, d'une huile de silicone, d'une cire, d'un alcool gras, d'un alcool de Guerbet ou de son ester, d'une substance active pharmaceutique fonctionnelle lipophile ou d'un mélange de ces substances, et
(d) de l'éthanol,
jusqu'à ce qu'il se forme un liquide transparent homogène, **caractérisée en ce que** le mélange est réalisé dans un milieu anhydre.

10. Préphase de nanodispersion selon la revendication 9, **caractérisée en ce que** le mélange est réalisé dans des dispositifs d'agitation habituels sans l'application de grandes forces de cisaillement ou de cavitation.

11. Utilisation d'une nanodispersion contenant
(a) 0,1% à 30% en poids d'un phospholipide,
(b) 1% à 50% en poids d'alcools gras polyéthoxylés, d'acides gras polyéthoxylés, de dérivés de vitamine E polyéthoxylée, de lanoline polyéthoxylée et de ses dérivés, de glycérides partiels d'acides gras polyéthoxylés, d'alkylphénols polyéthoxylés, d'hémi-esters d'acide sulfurique et d'alcools gras polyéthoxylés et de leurs sels, d'amines grasses et d'amides gras polyéthoxylés, d'hydrates de carbone polyéthoxylés, ou de polymères séquences d'oxyde d'éthylène et d'oxyde de propylène,
(c) 0,1% à 80% en poids d'un di- ou tri-glycéride naturel ou synthétique ou partiellement synthétique, d'une huile minérale, d'une huile: de silicone, d'une cire, d'un alcool gras, d'un alcool de Guerbet ou de son ester, d'une substance active pharmaceutique fonctionnelle lipophile ou d'un mélange de ces substances, et
(d) de l'éthanol,
dans des formulations pharmaceutiques finales, dans laquelle la nanodispersion peut être obtenue.
(α) en mélangeant les composants (a), (b), (c) et (d) dans des agitateurs habituels jusqu'à ce qu'il se forme un liquide transparent homogène, et
(β) en ajoutant le liquide obtenu à l'étape (α) dans la phase aqueuse des formulations pharmaceutiques finales, **caractérisée en ce que** les étapes (α) et (β) sont réalisées sans l'application de grandes forces de cisaillement ou de cavitation.

12. Utilisation selon la revendication 11, **caractérisée en ce que** l'étape (α) est réalisée dans un milieu anhydre.

13. Utilisation selon la revendication 11 ou 12, **caractérisée en ce que** l'étape (β) est réalisée sans homogénéisation.

14. Utilisation selon l'une quelconque des revendications 11 à 13, **caractérisée en ce que** les particules contenues dans la nanodispersion ont un diamètre moyen inférieur à 50 nm.

15. Utilisation selon l'une quelconque des revendications 11 à 14, **caractérisé en ce que** la formulation pharmaceutique finale est une préparation liquide, semi-solide ou solide.
